# EUROPEAN PATENT APPLICATION

(11) **EP 4 497 743 A1**
(43) Date of publication of application: **29.01.2025**
(21) Application number: 23382785.6
(22) Date of filing: 28.07.2023
(51) Int. Cl.: C07C 29/56, C07C 35/18, C07C 45/51, C07C 49/403, C07C 49/603, C07C 41/09, C07C 43/188, A01P 17/00, A01P 19/00

(54) **TRANSFORMATION OF THE PHEROMONE 3-METHYL-2-CYCLOHEXEN-1-OL IN THE PRESENCE OR ABSENCE OF [RUCLCP(PTA)2] AND [RUCP(H2O-KO)(PTA)2]CF3SO3**

(71) Applicant: Univerdidad De Almeria, 04120 Almeria (ES)
(72) Inventor: ROMEROSA NIEVAS, Antonio Manuel, 04120 Almería (ES); SCALAMBRA, Franco, 04120 Almería (ES); LÓPEZ SÁNCHEZ, María Belén, 04120 Almería (ES)
(74) Representative: Hoffmann Eitle

(57) **Abstract**

The present invention refers to the chemical field, in particular to homogeneous organometallic catalysis in water-rich media, more particularly to the transformation of the pheromone 3-methyl-2-cyclohexen-1-ol in the presence or absence of [RuClCp(PTA)₂] and [RuCp(H₂O-κO)(PTA)₂]CF₃SO₃

## Description

### FIELD OF THE INVENTION

The present invention refers to the chemical field, in particular to homogeneous organometallic catalysis in water-rich media, more particularly to the transformation of the pheromone 3-methyl-2-cyclohexen-1-ol in the presence or absence of [RuClCp(PTA)₂] and [RuCp(H₂O-κ*O*)(PTA)₂]CF₃SO₃

### BACKGROUND ART

Homogeneous organometallic catalysis in water-rich media is an interesting approach that may enable more sustainable and eco-friendly synthetic processes.¹⁻³ Among them, the isomerization of allylic alcohols is an interesting reaction that allows the obtention of carbonyl compounds in one pot and with 100% atom economy, bypassing the harsh conditions required with the non-catalytic methods (Scheme 1).^{4,5} In addition, the carbonyl compounds resulting from the isomerization of highly substituted allylic alcohols, such as terpenes and natural opioids, have significant potential in medicinal and fine chemistry.^{6,7}

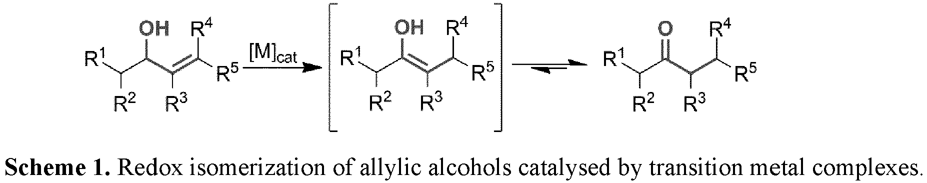

Water-soluble metal complexes useful as catalysts needs to be constituted by adequate ligands that stabilize the complex and provide solubility in water, such as hydrophilic tertiary phosphines.⁸⁻¹⁶ The most efficient metal complexes found to be active for the isomerization of allylic alcohols contain transition metals^{7,17,18} such as Fe,¹⁹⁻²¹ Rh,²²⁻²⁸ Ir,²⁹⁻³¹ Ni,³²⁻³⁴ Ru,³⁵⁻⁴⁰ Co,⁴¹ and Pd.⁴²

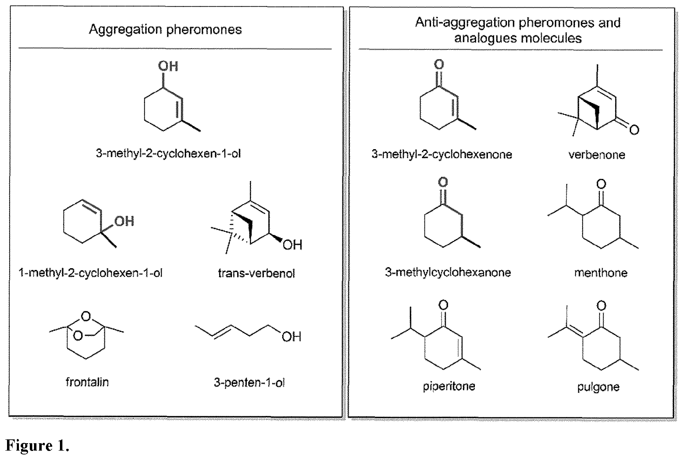

In the present invention, we have shown that the water-soluble complexes [RuClCp(PTA)₂] (**1**)⁷² and [RuCp(H₂O-κ*O*)(PTA)₂]CF₃SO₃ (**2**)⁷³ are active catalysts for the transformation of a variety of linear and cyclic allylic alcohols in water, including 3-methyl-2-cyclohexen-1-ol.⁷³⁻⁷⁷ The products obtained by the transformation of the abovementioned alcohol, together with the starting allylic alcohol itself, are pheromones involved in the natural aggregation/antiaggregation system of the Douglas-fir beetle (*Dendroctonus pseudotsugae*), known to be the major plague of coniferous trees.⁷⁸ The molecules 3-methyl-2-cyclohexen-1-ol and 1-methyl-2-cylohexen-1-ol are known aggregative pheromones, while 3-methyl-2-cyclohexenone and 3-methylcyclohexanone have an antiaggregative role (Figure 1). These have been used for decades to repel the Douglas-fir beetle.⁷⁹⁻⁸⁹

### SUMMARY OF THE INVENTION

A first aspect of the present invention refers to a process for the catalytic isomerization, oxidation, 1,3-transposition, and/or etherification of 3-methyl-2-cyclohexen-1-ol to 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or any mixture thereof, comprising:
a. providing a starting material or composition comprising 3-methyl-2-cyclohexen-1-ol;
b. contacting the starting material or composition with an aqueous and/or organic media;
c. carrying out a reaction in the aqueous and/or organic media resultant from step b) to produce 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or any mixture thereof; and
d. optionally recovering the 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or any mixture thereof;
wherein the reaction of step c) is performed in the aqueous or organic media of step b) during a time interval of between 1 minute and 7 days, preferably between 1 hour and 3 days, and wherein the reaction takes place at a pressure within the range of 10⁻² and 10 atmospheres (atm), preferably 1 atm, and at a temperature between 0.1°C and 150°C.

It is noted that the reaction of step c) is carried out during or subsequently to the contacting step b) in the aqueous and/or organic media resultant from step b).

In a preferred embodiment of the first aspect of the invention, step b) is carried out by contacting the starting material with the aqueous and/or organic media at a ratio of the aqueous and/or organic media to 3-methyl-2-cyclohexen-1-ol between 1:0.01 and 1:10000 v/v.

In another preferred embodiment of the first aspect of the invention, the aqueous and/or organic media is an aqueous media, preferably water, and the reaction of step c) takes place under aerobic conditions or under an inert atmosphere such as a nitrogen atmosphere, at a temperature between 30°C and 100°C, preferably about 70°C, and the concentration of 3-methyl-2-cyclohexen-1-ol used in contacting step b) is greater than 0.05 mM, preferably between 0.05 mM and 20 mM.

In another preferred embodiment of the first aspect of the invention, the concentration of 3-methyl-2-cyclohexen-1-ol used in contacting step b) is between 0.1 mM and 10 mM, preferably 0.9 mM.

In another preferred embodiment of the first aspect of the invention, the concentration of 3-methyl-2-cyclohexen-1-ol used in put in contact step b) is between 0.05 mM and 0.5 mM, preferably 0.2 mM.

In another preferred embodiment of the first aspect of the invention, the concentration of 3-methyl-2-cyclohexen-1-ol used in step b) is equal or greater than 0.6 mM, preferably equal or greater than 0.7 nM, preferably equal or greater than 0.8 nM, preferably equal or greater than 0.9 nM, preferably equal or greater than 1 nM, preferably equal or greater than 5 nM, preferably equal or greater than 10 nM.

In yet another preferred embodiment of the first aspect of the invention (referred to herein as "process of the invention with a catalyst"), the process comprises:
a. providing a starting material or composition comprising 3-methyl-2-cyclohexen-1-ol;
b. contacting or putting in contact the starting material or composition with a catalyst in an aqueous and/or organic media;
c. carrying out a reaction in the aqueous and/or organic media resultant from step b) to produce 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or any mixture thereof; and
d. optionally recovering the 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or any mixture thereof;
wherein the reaction of step c) is performed in the aqueous or organic media of step b) during a time interval of between 1 minute and 7 days, preferably between 1 hour and 3 days, and wherein the reaction takes place at a pressure within the range of 10⁻² and 10 atmospheres (atm), preferably 1 atm, and at a temperature between 0. 1°C and 150°C.

It is noted that the reaction of step c) is carried out during or subsequently to the contacting step b) in the aqueous and/or organic media resultant from step b).

In another preferred embodiment of the process of the invention with a catalyst, the catalyst is a Ruthenium complex. Preferably, the catalyst has a chemical structure of formula I: 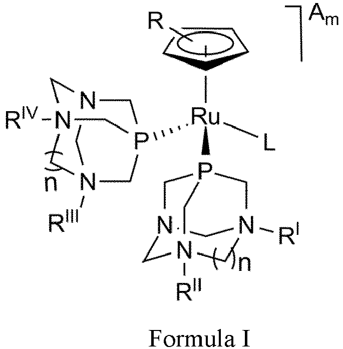 wherein:
- L is selected from fluoride, chloride, bromide, iodide, carbonyl, cyanide, hydride; azide, pyridine, pyrazine, pyrimidine, bipyridine, N-heterocyclic carbenes; alcohols such as methanol, isopropanol, ethanol, tert-butanol, n-butanol, and their alkoxides; oxobases such as phosphate, carbonate, sulfate, formate, and their acid derivatives; allylic alcohol η²-complexes;
- R is H or a methyl group (CH₃);
- RI, RII, RIII, RIV are independently selected from an oxygen atom, H, a methyl group (CH₃), a (CH₂)ₙ-CH₃ (wherein n is 1 - 20), an acetyl, allyl, butyl, phenyl, benzyl, nitrobenzyl, or dichloroallyl group;
- n is 0-1;
- wherein A is preferably selected from chloride, iodide, phosphate, triflate, sulfate, bromide, tetrafluoroborate, hexafluoroborate, tetrafluoromethane, tetrafluoroammonium, BArF; and
- m is 0 - 3.

More preferably, the catalyst is selected from the list consisting of the metal complexes [RuClCp(PTA)₂] (**1**) or [RuCp(OH₂)(PTA)₂]CF₃SO₃ (**2**) (PTA = 1,3,5-triaza-7-phosphaadamantane).

In a preferred embodiment of the process of the invention with a catalyst, the catalyst is [RuCp(OH₂)(PTA)₂]CF₃SO₃ (**2**), and the reaction takes place under an inert atmosphere such as a nitrogen atmosphere, at a temperature between 30°C and 100°C, preferably about 70°C, and the concentration of the catalyst used during step b) is equal or greater than 0.6 mol%, preferably equal or greater than 0.7 mol%, preferably equal or greater than 0.8 mol%, preferably equal or greater than 0.9 mol%, preferably equal or greater than 1 mol%, preferably equal or greater than 5 mol%, preferably equal or greater than 10 mol%. Preferably, the aqueous and/or organic media is water or an organic solvent or a mixture of water and an organic solvent such as cyclohexane, toluene, octane, heptane, hexane, pentane, benzene, xylene, or fluorated solvents, wherein preferably the mixture of water to organic solvent is present at a ratio of from 0.01:1 to 100:1 v/v.

In another preferred embodiment of the process of the invention with a catalyst, the catalyst is selected from the list consisting of the metal complexes [RuClCp(PTA)₂] (**1**) or [RuCp(OH₂)(PTA)₂]CF₃SO₃ (**2**) (PTA = 1,3,5-triaza-7-phosphaadamantane), and the reaction takes place under aerobic conditions and the concentration of the catalyst used during step b) is between 0.05 and 0.5 mol%, preferably about 0.1 mol%, and the aqueous media is water.

In another preferred embodiment of the process of the invention with a catalyst, the catalyst is [RuClCp(PTA)₂] (**1**), and the reaction takes place under an inert atmosphere such as a nitrogen atmosphere, at a temperature between 30°C and 100°C, preferably about 70°C, and the concentration of the catalyst used during step b) is equal or greater than 0.6 mol%, preferably equal or greater than 0.7 mol%, preferably equal or greater than 0.8 mol%, preferably equal or greater than 0.9 mol%, preferably equal or greater than 1 mol%, preferably equal or greater than 5 mol%, preferably equal or greater than 10 mol%. Preferably, the aqueous or organic media used during step b) is an aqueous media such as water or an organic solvent or a mixture of water and an organic solvent such as cyclohexane, toluene, octane, heptane, hexane, pentane, benzene, xylene, or fluorated solvents, wherein preferably the mixture of water to organic solvent is present at a ratio of from 0.01: 1 to 100:1 v/v.

In yet another preferred embodiment of the process of the invention with a catalyst, the concentration of 3-methyl-2-cyclohexen-1-ol used during step b) is greater than 0.05 mM, preferably between 0.05 mM and 20 mM. Preferably, the contacting step b) is between 0.1 mM and 10 mM, preferably 0.9 mM. Preferably, the concentration of 3-methyl-2-cyclohexen-1-ol used during step b) is between 0.05 mM and 0.5 mM, preferably 0.2 mM. Also preferably, the concentration of 3-methyl-2-cyclohexen-1-ol used during step b) is equal or greater than 0.6 mM, preferably equal or greater than 0.7 nM, preferably equal or greater than 0.8 nM, preferably equal or greater than 0.9 nM, preferably equal or greater than 1 nM, preferably equal or greater than 5 nM, preferably equal or greater than 10 nM.

In another preferred embodiment of the first aspect of the invention or of any of its preferred embodiments, the water/3-methyl-2-cyclohexen-1-ol molar ratio used during step b) is 100 or more, preferably 150 or more, preferably 170 or 176 or more.

In another preferred embodiment of the first aspect of the invention or of any of its preferred embodiments, the reaction process takes place at a pH between 3 and 9, preferably at about 8 or at about 4.5.

A second aspect of the invention refers to a composition comprising 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or a mixture thereof obtained by the process of the first aspect of the invention or of any of its preferred embodiments.

A third aspect of the invention refers to the use of the composition of the second aspect for the control of Douglas-fir beetle.

### BRIEF DESCRIPTION OF THE FIGURES

**Figure 1****.** Aggregation pheromones, antiaggregation pheromones and analogues molecules of the female Douglas-fir beetle (Dendroctonous).
**Figure 2****.** Conversion of 3-methyl-2-cyclohexen-1-ol into 3-methylcyclohexanone and 1-methyl-2-cyclohexen-1-ol catalyzed vs. mol of water/substrate proportion. Conditions: **1** and **2** (1 mol%), H₂O and CH₃OH, 2 h, 70°C, N₂ (a) and (c), air (b) and (d). Conversion % by GC.
**Figure 3****.** Conversion of 3-methyl-2-cyclohexen-1-ol into 3-methylcyclohexanone and 1-methyl-2-cyclohexen-1-ol in biphasic H₂O/cyclohexane 1: 1. Conditions: **2** (1 mol%) 70°C, H₂O (2.5 mL), cyclohexane (2.5 mL), N₂ (a), air (b). Conversion % by GC.
**Figure 4****.** Conversion vs. recycling runs (3 h each) of complex **2** in biphasic H₂O/cyclohexane. Conditions: **2** (1 mol%), H₂O (2.5 mL), cyclohexane (2.5 mL), 70°C, N₂. Conversion % by GC.

### DESCRIPTION OF EMBODIMENTS

For the present invention, we have studied the reactivity of the cyclic allylic alcohol 3-methyl-2-cyclohexen-1-ol, which is an important pheromone of the Douglas beetle, in the presence and absence of the metal complexes [RuClCp(PTA)₂] (1) and [RuCp(OH₂)(PTA)₂]CF₃SO₃ (**2**) (PTA = 1,3,5-triaza-7-phosphaadamantane). Both compounds catalyse the conversion of 3-methyl-2-cyclohexen-1-ol into 3-methylcyclohexanone in water, methanol and a biphasic mixture of water/cyclohexane. These reactions lead to the isomerization of 3-methyl-2-cyclohexen-1-ol into 3-methylcyclohexanone, oxidation into 3-methy-2-cyclohexenone and 1,3-transposition into 1-methyl-2-cyclohexen-1-ol.

More particularly, as shown in the examples of the present invention, complexes **1** and **2** proved to be efficient for the redox isomerization of the aggregative pheromone of the Douglas fir beetle 3-methyl-2-cyclohexen-1-ol to 3-methylcyclohexanone, which possesses antiaggregative behaviour. Also, we found that 3-methyl-2-cyclohexen-1-ol can be transformed into the 1,3-transposition product, the aggregative pheromone 1-methyl-2-cyclohexen-1-ol and into the oxidation product, the antiaggregative pheromone 3-methyl-2-cyclohexenone. The reaction path was determined by the catalyst ratio, the solvent, and the atmosphere in which the reaction is carried out. In pure water, a high catalyst loading (10 mol%) was able to transform 3-methyl-2-cyclohexen-1-ol mostly into 3-methyl-2-cyclohexenone under aerobic conditions (TON = 5.5, TOF = 0.23 h⁻¹), and using a lower proportion of catalyst (1 mol%) the substrate 3-methyl-2-cyclohexen-1-ol was mostly transformed into 3-methylcyclohexanone under anaerobic conditions (**1**: TON = 36, TOF = 7.2 h⁻¹; **2**: TON = 70, TOF = 14 h⁻¹), and into 1-methyl-2-cyclohexen-1-ol under aerobic conditions (approx. 40%). Furthermore, optimal catalytic conversion for the isomerization was achieved in presence of **2** and under inert conditions, with a water/substrate ratio of 176 (TON = 97, TOF = 48.5 h⁻¹), and in MeOH, where **2** was able to isomerize 97 eq. of substrate (TON = 97, TOF = 48.5 h⁻¹). Even more, **2** can isomerize 100 eq. of 3-methyl-2-cyclohexen-1-ol in biphasic cyclohexane/H₂O where 1 mol% of **2** was recycled up to 5 cycles (TON = 285, TOF = 57 h⁻¹). Its reactivity with 3-methyl-2-cyclohexen-1-ol was also investigated by NMR to reveal the catalytic mechanism. We identified the intermediate formed between **2** and the substrate, which evidence the η²-CH=CH coordination of the allylic alcohol to the metal center, and the formation of the metal hydride species [RuCpH(PTA)₂]⁺. Finally, the transformation of 3-methyl-2-cyclohexen-1-ol in water without catalyst evidenced the exclusive formation exclusively of 1-methyl-2-cyclohexen-1-ol.

In the present invention, we thus have demonstrated that different ratios of 3-methyl-2-cyclohexen-1-ol, 1-methyl-2-cyclohexen-1-ol, 3-methyl-2-cyclohexenone and 3-methylcyclohexanone, which are very important pheromones of Douglas beetle used to control this plague, can be obtained by using simple one-pot reactions, and just upon slight modifications of the reaction conditions.

Therefore, a first aspect of the invention refers to a process for the catalytic isomerization, oxidation, 1,3-transposition and/or etherification of 3-methyl-2-cyclohexen-1-ol (substrate) to 1-methyl-2-cyclohexen-1-ol, 3-methyl-2-cyclohexenone, 3-methylcyclohexanone, 1-methyl-3-(3-methyl-2-cyclohexen-1-yl)oxycyclohexene and/or any mixture thereof, comprising:
a. providing a starting material or composition comprising or consisting of 3-methyl-2-cyclohexen-1-ol;
b. contacting the starting material or composition with an aqueous and/or organic media;
c. carrying out a reaction in the aqueous and/or organic media resultant from step b), to produce 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or any mixture thereof; and
d. optionally recovering, isolating and/or purifying the 1-methyl-2-cyclohexen-1-ol, 3-methyl-2-cyclohexenone, 3-methylcyclohexanone, 1-methyl-3-(3-methyl-2-cyclohexen-1-yl)oxycyclohexene and/or any mixture thereof;
wherein the reaction of step c) is performed in the aqueous and/or organic media of step b), preferably water or any mixture thereof of water with an organic medium such as, but not limited to, ethanol, methanol, cyclohexane, or isopropanol, during a time interval of between 1 minute and 7 days, preferably between 1 hour and 3 days, and wherein the reaction takes place at a pressure within the range of 10⁻² and 10 atmospheres (atm), preferably at about 1 atm, and at a temperature between 0.1°C and 150°C, preferably between 30°C and 100°C, more preferably of about 70°C.

It is noted that the reaction of step c) is carried out during or subsequently to the contacting step b) in the aqueous and/or organic media resultant from step b).

It is herein noted that further organic solvents or media useful in the present invention can be selected such as those from selected from the list consisting of toluene, octane, heptane, hexane, pentane, benzene, xylene, fluorated solvent or any mixture thereof. Preferred aqueous and/or organic media wherein the reaction of step c) is carried out are water, methanol or a biphasic mixture of water/cyclohexane.

In another preferred embodiment of the first aspect of the invention, the process comprises the use of a catalyst (from hereinafter "process of the invention with a catalyst"), the process comprising the steps of:
a. providing a starting material or composition comprising or consisting of 3-methyl-2-cyclohexen-1-ol;
b. contacting the starting material or composition with a catalyst in an aqueous and/or organic media;
c. carrying out a reaction in the aqueous and/or organic media resultant from step b), to produce 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or any mixture thereof; and
d. optionally recovering, isolating and/or purifying the 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or any mixture thereof.
wherein the reaction of step c) is performed in the aqueous and/or organic media of step b), preferably water or any mixture thereof of water with an organic medium such as, but not limited to, ethanol, methanol, cyclohexane, or isopropanol, during a time interval of between 1 minute and 7 days, preferably between 1 hour and 3 days, and wherein the reaction takes place at a pressure within the range of 10⁻² and 10 atm, preferably at about 1 atm, and at a temperature between 0.1°C and 150°C, preferably between 30°C and 100°C, more preferably about 70°C.

It is noted that the reaction of step c) is carried out during or subsequently to the contacting step b) in the aqueous and/or organic media resultant from step b).

Preferred aqueous and/or organic media wherein the reaction of step c) is carry out are water, methanol or a biphasic mixture of water/cyclohexane.

It is herein again noted that further organic solvents or media useful in the present invention can be used such as those selected from the list consisting of toluene, octane, heptane, hexane, pentane, benzene, xylene, fluorated solvent or any mixture thereof.

In a preferred embodiment, the catalyst in the process of the invention with a catalyst is a Ruthenium complex. Preferably, the catalyst has the following chemical structure of formula I: 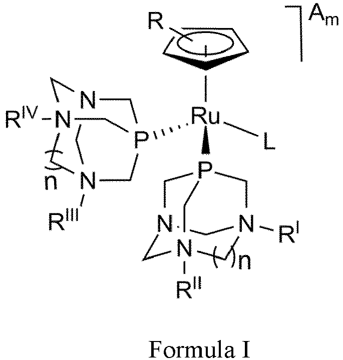 wherein:
- L can be selected from fluoride, chloride, bromide, iodide, carbonyl, cyanide, hydride; azide, pyridine, pyrazine, pyrimidine, bipyridine, N-heterocyclic carbenes; alcohols such as methanol, isopropanol, ethanol, tert-butanol, n-butanol, and their alkoxides; oxobases such as phosphate, carbonate, sulfate, formate, and their acid derivatives; and allylic alcohol η² complexes such as ([RuCp(η²-C₇H₁₂OH)])(PTA)₂]⁺);
- R is H or a methyl group (CH₃);
- RI, RII, RIII, RIV are independently selected from an oxygen atom, H, a methyl group (CH₃), a (CH₂)n-CH₃ (wherein n is 1 - 20), an acetyl, allyl, butyl, phenyl, benzyl, nitrobenzyl, or dichloroallyl group;
- n is 0-1;
- wherein A is preferably selected from chloride, iodide, phosphate, triflate, sulfate, bromide, tetrafluoroborate, hexafluoroborate, tetrafluoromethane, tetrafluoroammonium, BAr^{F} (tetrakis[3,5-bis(trifluoromethyl)phenyl]borate); and
- m is 0-3

In a preferred embodiment, the catalyst of the process of the invention with a catalyst is selected from the list consisting of the metal complexes [RuClCp(PTA)₂] (1) or [RuCp(OH₂)(PTA)₂]CF₃SO₃ (2) (PTA = 1,3,5-triaza-7-phosphaadamantane).

In a preferred embodiment of the first aspect of the invention (including the process of the invention with a catalyst), step b) is carried out by contacting the starting material or composition comprising or consisting of 3-methyl-2-cyclohexen-1-ol with the aqueous and/or organic media, optionally comprising the catalyst, at a molar ratio of the aqueous and/or organic media to 3-methyl-2-cyclohexen-1-ol (substrate) of between 1:0.01 -1:10000 v/v, wherein preferred molar ratios are approx. 1:0.01 v/v (an aqueous media to 3-methyl-2-cyclohexen-1-ol molar ratio of 100), 0.5:0.3 v/v (an aqueous media to 3-methyl-2-cyclohexen-1-ol molar ratio of approx. 66), or 0.3:0.1 v/v (an aqueous media to 3-methyl-2-cyclohexen-1-ol molar ratio of approx. 3). Preferably, the aqueous media to 3-methyl-2-cyclohexen-1-ol molar ratio during step is 100 or more, preferably 150 or more, preferably 170 or 176 or more. Preferably, the aqueous media to 3-methyl-2-cyclohexen-1-ol molar ratio during step is in the range of from 100 to 1000, preferably in the range of from 150 to 1000, preferably in the range of from 170 to 1000. Preferably, all of the values indicated herein referring to the molar ratios of aqueous media, preferably water, to 3-methyl-2-cyclohexen-1-ol (substrate) are deemed to be approximate. The term "approximate" as used herein shall be herein understood as +/- 30%, +/-25%, +/- 20%, +/- 15%, +/- 10%, +/- 5%, or +/- 1% of any of the indicated values. For example, when referring to a molar ratio of approx. 0.5:0.3 v/v, it is herein understood to encompass 0.45:0.33 (as each of these values can be understood to be a +/- 10% approximation). It is herein also understood that the term "approximate" or "approx." can also be used to refer to the exact indicated value. Furthermore, step b) can be carried out by contacting the starting material or composition comprising or consisting of 3-methyl-2-cyclohexen-1-ol with the aqueous and/or organic media comprising the catalyst. For this specific case, all of the above molar ratios applied and, in addition, for this case, the catalyst can be present at a concentration in the aqueous and/or organic media equal or greater than 0.6 mol%, preferably equal or greater than 0.7 mol%, preferably equal or greater than 0.8 mol%, preferably equal or greater than 0.9 mol%, preferably equal or greater than 1 mol%, preferably equal or greater than 5 mol%, preferably equal or greater than 10 mol%. The catalyst can also be present at a concentration in the aqueous and/or organic media between 0.05 and 0.5 mol%, preferably about 0.1 mol%.

In another preferred embodiment of the first aspect of the invention (including the process of the invention with a catalyst), the aqueous media is water or any mixture thereof of water with an organic medium such as, but not limited to, ethanol, methanol, cyclohexane, or isopropanol; and the reaction step takes place either under aerobic conditions or under an inert atmosphere such as a nitrogen atmosphere, the temperature of the reaction is preferably between 30°C and 100°C, preferably about 70°C, and the concentration of 3-methyl-2-cyclohexen-1-ol during the contacting step of step b) is greater than about 0.05 mM, preferably between about 0.05 mM and about 20 mM. Preferably, the concentration of 3-methyl-2-cyclohexen-1-ol during step is between about 0.1 mM and about 10 mM. Preferably, the concentration of 3-methyl-2-cyclohexen-1-ol during the contacting step is between about 0.05 mM and about 0.5 mM. Still preferably, the concentration of 3-methyl-2-cyclohexen-1-ol is equal or greater than 0.6 mM, preferably equal or greater than 0.7 nM, preferably equal or greater than 0.8 nM, preferably equal or greater than 0.9 nM, preferably equal or greater than 1 nM, preferably equal or greater than 5 nM, preferably equal or greater than 10 nM. The term "about" as used herein shall be herein understood as +/- 30%, +/- 25%, +/- 20%, +/- 15%, +/- 10%, +/- 5%, or +/- 1% of any of the indicated values. It is herein also understood that the term "about" can also be used to refer to the exact indicated value. On the other hand, as indicated above, the aqueous media during the contacting step can be selected from any of the molar ratios of aqueous media, preferably water, to 3-methyl-2-cyclohexen-1-ol (substrate) indicated above. Preferably, the aqueous media to 3-methyl-2-cyclohexen-1-ol molar ratio is 100 or more, preferably 150 or more, preferably 170 or 176 or more. Preferably, the aqueous media to 3-methyl-2-cyclohexen-1-ol molar ratio is in the range of from 100 to 1000, preferably in the range of from 150 to 1000, preferably in the range of from 170 to 1000. Furthermore, as already indicated, if a catalyst is present, such catalyst can be present at a concentration in the aqueous media equal or greater than 0.6 mol%, preferably equal or greater than 0.7 mol%, preferably equal or greater than 0.8 mol%, preferably equal or greater than 0.9 mol%, preferably equal or greater than 1 mol%, preferably equal or greater than 5 mol%, preferably equal or greater than 10 mol%. The catalyst can also be present at a concentration in the aqueous media between 0.05 and 0.5 mol%, preferably about 0.1 mol%.

In a preferred embodiment, the catalyst of the process of the invention with a catalyst is [RuCp(OH₂)(PTA)₂]CF₃SO₃ (2), and the reaction of step c) takes place under an inert atmosphere such as a nitrogen atmosphere, preferably in a temperature between 30°C and 100°C, preferably about 70°C, and during the contacting step, a concentration of the catalyst equal or greater than 0.6 mol%, preferably equal or greater than 0.7 mol%, preferably equal or greater than 0.8 mol%, preferably equal or greater than 0.9 mol%, preferably equal or greater than 1 mol%, preferably equal or greater than 5 mol%, preferably equal or greater than 10 mol%, is used. Preferably, the aqueous media used in the contacting step and the immediate or subsequent reaction is water or an organic solvent or a mixture of water and an organic solvent such as cyclohexane, wherein preferably the mixture of the aqueous media to organic solvent is present at a ratio of from 0.01:1 to 100:1 v/v. It is herein again noted that further organic solvents or media useful in the present invention can be selected from the list consisting of toluene, octane, heptane, hexane, pentane, benzene, xylene, fluorated solvent or any mixture thereof.

In another preferred embodiment, the catalyst of the process of the invention with a catalyst is selected from the list consisting of the metal complexes [RuClCp(PTA)₂] (1) or [RuCp(OH₂)(PTA)₂]CF₃SO₃ (2) (PTA = 1,3,5-triaza-7-phosphaadamantane), and the reaction of step c) takes place under aerobic conditions and the concentration of the catalyst used during the contacting step, is between 0.05 and 0.5 mol%, preferably about 0.1 mol%, and the aqueous media used during the contacting step and the immediate or subsequent reaction is water. Preferably, during the contacting step, the mixture of the aqueous media to organic solvent is present at a ratio of from 0.01:1 to 100:1 v/v. It is herein again noted that further organic solvents or media useful in the present invention can be selected from the list consisting of toluene, octane, heptane, hexane, pentane, benzene, xylene, fluorated solvent or any mixture thereof.

In another preferred embodiment, the catalyst of the process of the invention with a catalyst is [RuClCp(PTA)₂] (1), the reaction of step c) takes place under an inert atmosphere such as a nitrogen atmosphere, the temperature is between 30°C and 100°C, preferably about 70°C, and the concentration of the catalyst used during the contacting step is equal or greater than 0.6 mol%, preferably equal or greater than 0.7 mol%, preferably equal or greater than 0.8 mol%, preferably equal or greater than 0.9 mol%, preferably equal or greater than 1 mol%, preferably equal or greater than 5 mol%, preferably equal or greater than 10 mol%. Preferably, the aqueous media used during the contacting step and the immediate or subsequent reaction is water or an organic solvent or a mixture of water and an organic solvent such as cyclohexane, wherein preferably the mixture of water to organic solvent is present at a ratio of from 0.01: 1 to 100:1 v/v. It is herein again noted that further organic solvents or media useful in the present invention can be selected from the list consisting of toluene, octane, heptane, hexane, pentane, benzene, xylene, fluorated solvent or any mixture thereof.

In a preferred embodiment of the first aspect of the invention, the reaction process takes place at a pH between 3 and 9, preferably at about 8 or at about 4.5.

It is herein noted that in an alternative aspect of the first aspect of the invention, the contacting step b) of the process (including the process of the invention with a catalyst), can be carried out without an aqueous medium, in which case, the substrate shall act as the dissolvent, preferably when the process of the invention is carried out with a catalyst as defined in the present invention. Such a process without an aqueous media shall, according to the present invention, provide 1-methyl-2-cyclohexen-1-ol.

A second aspect of the invention refers to a composition comprising 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or a mixture thereof obtained by the process of the first aspect of the invention or of any of its preferred embodiments.

A third aspect of the invention refers to the use (non-therapeutic use) of the composition of the second aspect of the invention for the control of the Douglas-fir beetle.

The following examples are merely illustrative of the present invention and do not limit the same.

### EXAMPLES

### Example 1.

Initially, as a first evaluation step, the cyclic allylic alcohol 3-methy-2-cyclohexen-1-ol was kept with **1** (10 mol%) in water at 70°C, under air, for 24 h. In contrast to our initial expectations, the products obtained were a mixture of the expected isomerization product, 3-methyl-2-cyclohexenone, but only in a proportion of 14%, together with 1-methyl-2-cyclohexen-1-ol, in a higher proportion of 34%, which is the result of the 1,3-transposition of the substrate, and 3-methylcyclohexanone in a small amount of 4%, which is the oxidation product. Interestingly, after 72 h, the conversion into the oxidation product raised to 55%, whereas both isomerization and 1,3-transposition reached 7%. The use of air as economical stoichiometric oxidant enables the development of eco-friendly catalytic methods for the oxidation reaction of alcohols to carbonyl compounds, which is an important reaction in pharmaceutical and fragrances chemistry,^{58, 59 60-62} where the catalysts usually contain metals such as Fe,⁶³ Ru,⁶⁴ Ir,⁶⁵ Cu,⁶⁶ Rh,⁶⁷ V⁶⁸ and Pd,⁶⁹ used together with oxidants such as a peroxide or dioxygen.^{70,71}

Upon decreasing the catalyst loading to 1 mol% of complex 1 and after 5 h of reaction, different mixture of products was obtained depending on the reaction atmosphere: under nitrogen, the isomerization and 1,3-transposition products were obtained in a similar proportion (3-methylcyclohexanone: 36%; 1-methyl-2-cyclohexen-1-ol: 33%), whereas under air, the substrate is converted mainly into the 1,3-transposition (38%) vs. 4% of the isomerization product. In contrast, when 1 mol% of complex **2** was used under N₂, the main obtained product is that afforded by the isomerization reaction (70%) with only 18% of that obtained by 1,3-transposition. Similarly to when performed in the presence of complex **1** (1 mol%), the reaction under air with 1 mol% of **2** provided the isomerization product with only 7% conversion, but the results of the 1,3-transposition with 38% conversion.

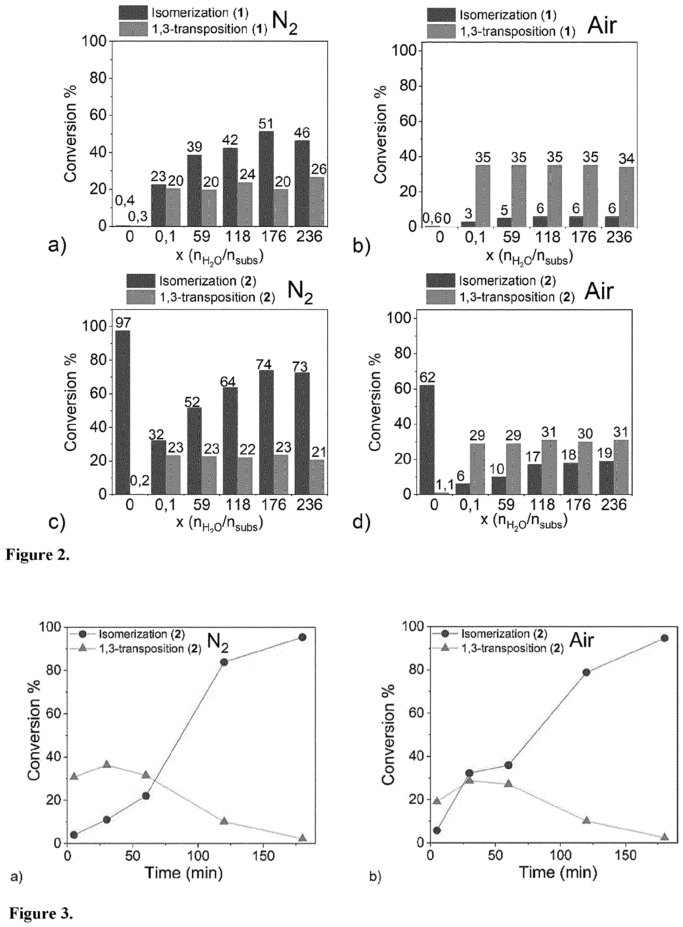

It was found also that resulting products are sensitive to the amount of water in the reaction. Indeed, in dry methanol and under a nitrogen atmosphere complex **1** was practically ineffective as a reaction catalyst, but the addition of water activates the substrate conversion, reaching after 2 h of reaction the maximum transformation into 3-methylcyclohexanone (51%) when water/substrate molar ratio of 176 was used (Figure 2.a). It is important to point out that the formation of the 1,3-transposition product requires the presence of a minimum amount of water; however, the conversion is not affected by the water/substrate ratio and did not exceed the 20% with the addition of a higher amount of water (Figure 2.a and 2.b). When **2** is used instead of **1** under the same reaction conditions, a substantially different ratio of products is obtained. When the reaction was carried out under nitrogen in methanol, almost complete isomerization after 2 h of the substrate (97%) was achieved, whereas in water the maximum conversion for the isomerization product was obtained when a water/substrate molar ratio of 176 was used, with a conversion of 74% after 2 h, and 85% after 5 h (Figure 2.c).

When the reaction was carried out under air and in methanol, only complex **2** is able to catalyse the transformation of the substrate, forming exclusively the isomerization product with 62% conversion after 2 h. Whereas under air and in water, the proportions of the products obtained were clearly different with respect to the anaerobic conditions, regardless of the catalyst used. A marked decrease in the isomerization product is observed, together with a slight increment in the 1,3-transposition product, that sets at around the 35% and does not change upon increasing the amount of water in the medium (Figure 2.b and 2.d).

Reactions in water at pH = 4.5, 7 and 8 were also studied. The results obtained with both complexes **1** and **2** as catalysts under nitrogen were similar: the best isomerization rate was reached after 2 h of reaction at pH = 8, being **2** more active than **1** (**1**: 38%; **2**: 55%). At pH = 7 a drastic drop in catalytic activity of both complexes to 2-3% is observed. Under air, neither **1** or **2** are significantly active for the isomerization reaction, obtaining after 2 h only the 8% and 5% of 3-methylcyclohexanone, respectively. It is important to point out that when isomerization drops, 1,3-transposition rises, both under nitrogen and under air. Comparing the isomerization conversion values obtained for each pH tested and in pure water, the results in pure water are clearly higher.

The last reactions performed in pure water involved the study of the transformation of 3-methyl-2-cyclohexen-1-ol in the presence of a lower proportion of complexes **1** and **2.** Reactions were carried out with 0.01 mol% and 0.1 mol% of **1** and **2,** at 70°C, both under nitrogen and air. The reactions performed prompt the exclusive formation of 1-methyl-2-cyclohexen-1-ol the 1,3-transposition product, giving a similar conversion (approx. 44% after 24 h) independently of the used catalyst and of the atmosphere. Only a small amount of isomerization product is obtained when using 0.1 mol% of **2** (N₂: 2%, Air: 1%, after 24 h).

Next, we started the study of the transformation of this substrate in the presence of both catalysts in biphasic systems in H₂O/cyclohexane 1: 1 at 70°C both under N₂ and air. One of the advantages of the biphasic reactions is the recyclability of the catalyst. In this regard, we wanted to test whether it was possible to reuse the aqueous phase containing **1** and **2** of the biphasic reaction. The results shows that the substrate 3-methyl-2-cyclohexen-1-ol is catalytically transformed by **1** (1 mol %) in the same mixture of products obtained in pure water, even obtaining a similar conversio. While complex **2** (1 mol %) is remarkably able to catalyse the isomerization of the substrate reaching higher conversion rates biphasic conditions than in pure water (Figure 3). Under these conditions, almost complete substrate conversion mediated by **2** was obtained after 3 h (96%). As soon as the reaction starts, the 1,3-transposition product predominates (36% after 30 min), but decreases progressively over time, reaching 2% at 3 h. Interestingly, in the biphasic system employed, the activity of complex **2** does not depend on the atmosphere, and almost identical conversions are reached under both aerobic and anaerobic conditions (Figure 3).

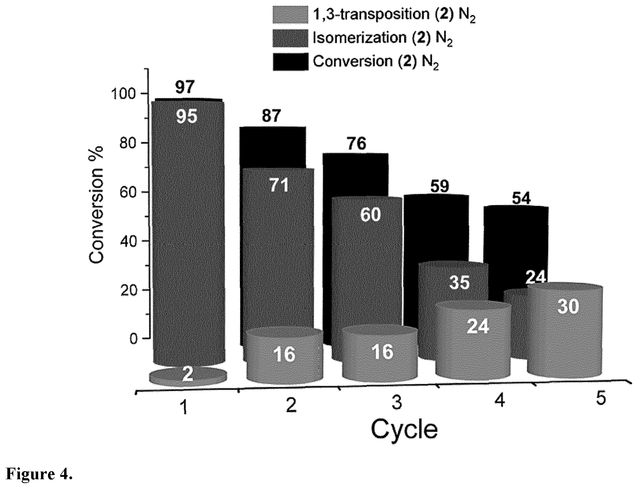

Therefore, in order to achieve the proposed objective, recyclability of **2** was evaluated under N₂ and for 3 h, providing better conversion, where the reuse of the aqueous phase containing **2** of the biphasic reaction was achieved for up to 5 consecutives 3 h runs, reaching a total TON of 285 equivalents of 3-methyl-2-cyclohexen-1-ol under N₂ (TOF_{5 cycles} = 19 h⁻¹). As shown in Figure 4, after the third cycle the conversion rate is considerably lower and after the fifth it drops to 24 %, which may be explained by the leaching of the catalyst from water to the organic phase. The decrease of the isomerization after each cycle, is also accompanied by a progressive increase of the 1,3-transposition product. Despite this fact, with the experiments performed here and at the best of our knowledge, ^{90,91} complex **2** is the best catalyst for the isomerization of 3-methyl-2-cyclohexen-1-ol, which is in agreement with the good activity of **2** found for isomerization of 2-cyclohexen-1-ol.⁷⁶

Finally, the reactivity of 3-methyl-2-cyclohexen-1-ol in pure water, without a catalyst, was tested. When the concentration of substrate was 10% v/v at 70 °C and under air and after 2 h, obtained products were 1-methyl-3-(3-methyl-2-cyclohexen-1-yl)oxycyclohexene (25%) and 1-mehyl-2-cyclohexen-1-ol (18 %), regardless of the atmosphere. Interestingly, upon lowering the concentration of substrate to 1% v/v, the 1,3-transposition compound 1-methyl-2-cyclohexen-1-ol was detected as the unique product (37%) together with the substrate, both under air and nitrogen. At lower reaction temperature (30°C) the 1,3-transposition product was also the unique observed product but in lower conversion (20%). This study suggests that the 1,3-transposition reaction appears to be mediated by water rather than the catalysts. Other important examples of this reaction were reported, such as the rearrangement of the vitamin A⁴³ and of linalool.⁴⁴ Usually, the catalytic 1,3-transposition of allylic alcohols,⁵⁰ is catalysed by transition metal oxo-complexes in high-oxidation states⁴⁵⁻⁴⁹ based on molybdenum,⁵⁰ wolframium,⁵¹ vanadium,^{52,53} ruthenium⁵⁴ or rhenium,⁵⁵⁻⁵⁷ but also different studies on the water-mediated 1,3-transposition of several allylic alcohols have been reported.⁹² Nevertheless this is the first example of the 1,3-transposition of 3-methyl-2-cyclohexen-1-ol in pure water, giving the expensive and important pheromone 1-methyl-2-cyclohexen-1-ol.

As a summary, Scheme 2 and Table 1 show the most relevant conversion data for the transformations of 3-methyl-2-cyclohexen-1-ol into 3-methylcyclohexanone (I), 3-methyl-2-cyclohexenone (II), 1-methyl-2-cyclohexen-1-ol (III), and 1-methyl-3-(3-methyl-2-cyclohexen-1-yl)oxycyclohexene (IV) under the different conditions studied.

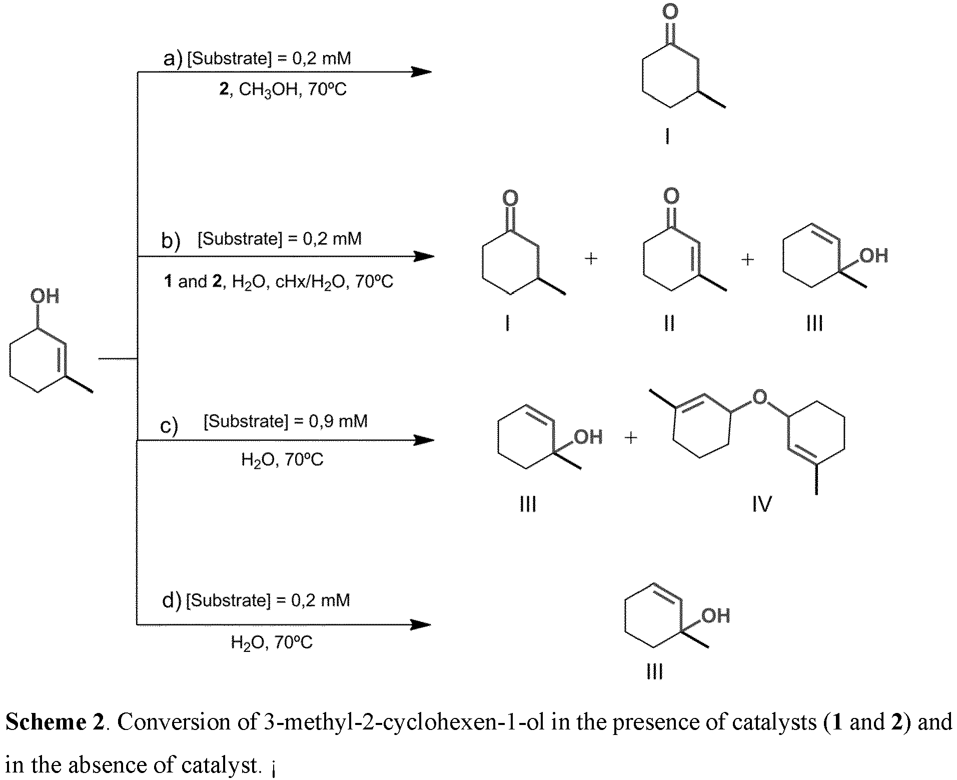

**Table 1. Transformation of 3-methyl-2-cyclohexen-1-ol under different reaction conditions and in presence of 1 and 2.**

| 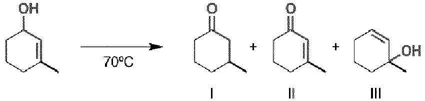 | | | | | | | | | | | | |
|---|---|---|---|---|---|---|---|---|---|---|---|---|
| **Catalyst** | **[Ru] mol%** | **t (h)** | **Solvent** | **Atmosphere** | **TON** | | **TOF** | | **I %** | **II %** | **III %** | **Conv. (%) ^{c}** |
| | | | | | **I** | **II** | **I** | **II** | | | | |
| **1** | 10 | 72 | 1 mL H₂O | Air | 0.7 | 6 | 0.01 | 0.2 | 7 | 55 | 7 | 69 |
| **1** | 1 | 5 | 1 mL H₂O | N₂ | 38 | - | 7 | - | 38 | - | 33 | 71 |
| **1** | 1 | 5 | 1 mL H₂O | Air | 4 | - | 0.8 | - | 4 | - | 38 | 42 |
| **1** | 1 | 2 | 2.5 mL H₂O | N₂ | 46 | - | 23 | - | 46 | - | 26 | 73 |
| **1** | 0.1 | 24 | 1 mL H₂O | Air | - | - | - | - | - | - | 48 | 48 |
| **1** | 1 | 5 | H₂O/cHx^{a} | N₂ | 45 | - | 22.5 | - | 45 | - | 22 | 67 |
| **1** | 1 | 5 | H₂O/cHx^{a} | Air | 18 | - | 9 | - | 18 | - | 37 | 55 |
| **2** | 1 | 5 | 1 mL H₂O | N₂ | 70 | - | 14 | - | 70 | - | 18 | 87 |
| **2** | 1 | 5 | 1 mL H₂O | Air | 7 | - | 1,4 | - | 7 | - | 39 | 46 |
| **2** | 1 | 2 | 2.5 mL H₂O | N₂ | 73 | - | 36 | - | 73 | - | 21 | 93 |
| **2** | 1 | 2 | 2.5 mL H₂O | Air | 19 | - | 9.5 | - | 19 | - | 31 | 50 |
| **2** | 1 | 5 | 2.5 mL H₂O | N₂ | 85 | - | 17 | - | 85 | - | 13 | 98 |
| **2** | 1 | 24 | 2.5 mL H₂O | N₂ | 94 | - | 3.9 | - | 94 | - | 3 | 97 |
| **2** | 0.1 | 24 | 1 mL H₂O | Air | | - | | - | | - | 38 | 38 |
| **2** | 1 | 2 | 0.5 mL CH₃OH | N₂ | 98 | - | 49 | - | 98 | - | 0.2 | 98 |
| **2** | 1 | 2 | 0.5 mL CH₃OH | N₂ | 98 | - | 49 | - | 98 | - | 0.2 | 98 |
| **2** | 1 | 3 | H₂O/cHx^{a} | N₂ | 95 | - | 32 | - | 95 | - | 2 | 97 |
| **2** | 1 | 15 | H₂O/cHx^{a} | N₂ | 285^{b} | - | 57 | - | 57 | - | 80 | 74.6 |

| | | | | | | | | | | | | |
|---|---|---|---|---|---|---|---|---|---|---|---|---|
| 70°C; I = 3-methylcyclohexanone, II = 3-methyl-2-cyclohexenone, III = 1-methyl-2-cyclohexen-1-ol; " 5 mL H₂O/Cyclohexane (1:1) (H₂O/cHx); ^{b} TON after 5 cycles under N₂ (H₂O/cHx), 1 mol % [Ru], 3 h/run). ^{c} % Measured by GC. | | | | | | | | | | | | |

### REFERENCES

(1) Cornils, B.; Herrmann, W. A. Aqueous-Phase Organometallic Catalysis: Concepts and Applications, 2nd Edition | Wiley. 2004**.**
(2) Duca, G. Homogeneous Catalysis with Metal Complexes. 2012, 102. https://doi.org/10.1007/978-3-642-24629-6.
(3) van Leeuwen, P. W. N. M. Homogeneous Catalysis Understanding the Art. 2004**.**
(4) Scalambra, F.; Lorenzo-Luis, P.; de los Rios, I.; Romerosa, A. Isomerization of Allylic Alcohols in Water Catalyzed by Transition Metal Complexes. Coord Chem Rev 2019, 393, 118-148. https://doi.org/10.1016/J.CCR.2019.04.012.
(5) van der Drift, R. C.; Bouwman, E.; Drent, E. Homogeneously Catalysed Isomerisation of Allylic Alcohols to Carbonyl Compounds. J Organomet Chem 2002, 650 (1-2), 1-24. https://doi.org/10.1016/S0022-328X(02)01150-6.
(6) Gómez, A. B.; Holmberg, P.; Bäckvall, J. E.; Martin-Matute, B. Transition Metal-Catalyzed Redox Isomerization of Codeine and Morphine in Water. RSC Adv 2014, 4 (74), 39519-39522. https://doi.org/10.1039/C4RA07735K.
(7) Cahard, D.; Gaillard, S.; Renaud, J. L. Asymmetric Isomerization of Allylic Alcohols. Tetrahedron Lett 2015, 56 (45), 6159-6169. https://doi.org/10.1016/J.TETLET.2015.09.098.
(8) Herrmann, W. A.; Kohlpaintner, C. W. Water-Soluble Ligands, Metal Complexes, and Catalysts: Synergism of Homogeneous and Heterogeneous Catalysis. Angewandte Chemie International Edition in English 1993, 32 (11), 1524-1544. https://doi.org/10.1002/anie.199315241.
(9) Ahrland, S.; Chatt, J.; Davies, N. R.; Williams, A. A. 55. The Relative Affinities of CoOrdinating Atoms for Silver Ion. Part II. Nitrogen, Phosphorus, and Arsenic. Journal of the Chemical Society (Resumed) 1958, 276-288. https://doi.org/10.1039/JR9580000276.
(10) Mena-Cruz, A.; Lorenzo-Luis, P.; Romerosa, A.; Serrano-Ruiz, M. Water-Soluble 3,7-Dimethyl-1,3,7-Triaza-5-Phosphabicyclo[3.3.1]Nonane (DmoPTA) as a Polydentate Ligand: Synthesis of [RuClCp(PPh 3 )-µ-DmoPTA-1κ P :2κ 2 N , N '-Co(Acac-κ 2 O , O ') 2 ]·H 2 O. Inorg Chem 2008, 47 (7), 2246-2248. https://doi.org/10.1021/ic702239w.
(11) Mena-Cruz, A.; Lorenzo-Luis, P.; Romerosa, A.; Saoud, M.; Serrano-Ruiz, M. Synthesis of the Water Soluble Ligands DmPTA and DmoPTA and the Complex [RuClCp(HdmoPTA)(PPh 3 )](OSO 2 CF 3 ) (DmPTA = N , N '-Dimethyl-1,3,5-Triaza-7-Phosphaadamantane, DmoPTA = 3,7-Dimethyl-1,3,7-Triaza-5-Phosphabicyclo[3.3.1]Nonane, HdmoPTA = 3,7-H-3,7-Dimethyl-1,3,7-Triaza-5-Phosphabicyclo[3.3.1]Nonane). Inorg Chem 2007, 46 (15), 6120-6128. https://doi.org/10.1021/ic070168m.
(12) Phillips, A. D.; Gonsalvi, L.; Romerosa, A.; Vizza, F.; Peruzzini, M. Coordination Chemistry of 1,3,5-Triaza-7-Phosphaadamantane (PTA): Transition Metal Complexes and Related Catalytic, Medicinal and Photoluminescent Applications. Coord Chem Rev 2004, 248 (11-12), 955-993. https://doi.org/10.1016/J.CCR.2004.03.010.
(13) Bravo, J.; Bolaño, S.; Gonsalvi, L.; Peruzzini, M. Coordination Chemistry of 1,3,5-Triaza-7-Phosphaadamantane (PTA) and Derivatives. Part II. The Quest for Tailored Ligands, Complexes and Related Applications. Coord Chem Rev 2010, 254 (5-6), 555-607. https://doi.org/10.1016/J.CCR.2009.08.006.
(14) Guerriero, A.; Peruzzini, M.; Gonsalvi, L. Coordination Chemistry of 1,3,5-Triaza-7-Phosphatricyclo[3.3.1.1]Decane (PTA) and Derivatives. Part III. Variations on a Theme: Novel Architectures, Materials and Applications. Coord Chem Rev 2018, 355, 328-361. https://doi.org/10.1016/J.CCR.2017.09.024.
(15) Luca, O. R.; Crabtree, R. H. Redox-Active Ligands in Catalysis. Chem Soc Rev 2013, 42 (4), 1440-1459. https://doi.org/10.1039/C2CS35228A.
(16) Fekete, M.; Joó, F. Redox Isomerization of Allylic Alcohols in Aqueous-Organic Biphasic Systems Catalyzed by Water-Soluble Ru(II)-N-Heterocyclic Carbene Complexes. Catal Commun 2006, 7 (10), 783-786. https://doi.org/10.1016/J.CATCOM.2006.03.001.
(17) Uma, R.; Crévisy, C.; Grée, R. Transposition of Allylic Alcohols into Carbonyl Compounds Mediated by Transition Metal Complexes. Chem Rev 2002, 103 (1), 27-51. https://doi.org/10.1021/CR0103165.
(18) Zhang, X.; Zhang, Y.; Liao, L.; Gao, Y.; Su, H. E. M.; Yu, J. Catalytic Asymmetric Isomerization of (Homo)Allylic Alcohols: Recent Advances and Challenges. ChemCatChem 2022. https://doi.org/10.1002/CCTC.202200126.
(19) Cherkaoui, H.; Soufiaoui, M.; Grée, R. From Allylic Alcohols to Saturated Carbonyls Using Fe(CO)5 as Catalyst: Scope and Limitation Studies and Preparation of Two Perfume Components. Tetrahedron 2001, 57 (12), 2379-2383. https://doi.org/10.1016/S0040-4020(01)00114-4.
(20) Iranpoor, N.; Mottaghinejad, E. Dodecacarbonyl Triiron, an Efficient Catalyst for Photochemical Isomerization of Unsaturated Alcohols, Ethers and Ester to Their Corresponding Carbonyl Compounds, Enol Ethers and Esters. J Organomet Chem 1992, 423 (3), 399-404. https://doi.org/10.1016/0022-328X(92)83133-3.
(21) Iranpoor, N.; Imanieh, H.; Iran, S.; Forbes, E. J. An Improved Method for the Efficient Conversion of Unstaurated Alcohols, Ethers and Esters to Their Corresponding Aldehydes, Ketones, Enol Ethers and Enol Esters. http://dx.doi.org/10.1080/00397918908052689 2006, 19 (17), 2955-2961. https://doi.org/10.1080/00397918908052689.
(22) Catalytic Production of Simple Enols Illustration of the Postulated Mechanism of Double-Bond Mi-Gration in the Formation of Enols from Allylic Alcohols (Sol = Solvent). and Thermodynamic Parameters for a Wide Range of Simple Enols.
(23) Bianchini, C.; Meli, A.; Oberhauser, W. Isomerization of Allylic Alcohols to Carbonyl Compounds by Aqueous-Biphase Rhodium Catalysis. New Journal of Chemistry 2000, 25 (1), 11-12. https://doi.org/10.1039/B001478H.
(24) Czégéni, C. E.; Fekete, M.; Tóbiás, E.; Udvardy, A.; Horváth, H.; Papp, G.; Joó, F. Redox Isomerization of Allylic Alcohols Catalyzed by New Water-Soluble Rh(I)-n-Heterocyclic Carbene Complexes. Catalysts 2020, 10 (11), 1-18. https://doi.org/10.3390/CATAL10111361.
(25) Tanaka, K.; Qiao, S.; Tobisu, M.; Lo, M. M. C.; Fu, G. C. Enantioselective Isomerization of Allylic Alcohols Catalyzed by a Rhodium/Phosphaferrocene Complex [18]. J Am Chem Soc 2000, 122 (40), 9870-9871. https://doi.org/10.1021/JA002471R/SUPPL_FILE/JA002471R_S.PDF.
(26) Ahlsten, N.; Lundberg, H.; Martín-Matute, B. Rhodium-Catalysed Isomerisation of Allylic Alcohols in Water at Ambient Temperature. Green Chemistry 2010, 12 (9), 1628-1633. https://doi.org/10.1039/C004964F.
(27) de Bellefon, C.; Caravieilhes, S.; Kuntz, mile G. Efficient Catalytic Isomerization of Allylic Alcohols to Carbonyl Compounds with Water Soluble Rhodium Complexes. Chimie / Chemistry 2000, 3, 607-614.
(28) Hiroya, K.; Kurihara, Y.; Ogasawara, K. Asymmetrization of Meso 1,4-Enediol Ethers by Isomerization with a Chiral Binap-RhI Catalyst. Angewandte Chemie International Edition in English 1995, 34 (20), 2287-2289. https://doi.org/10.1002/ANIE.199522871.
(29) Mantilli, L.; Mazet, C. Iridium-Catalyzed Isomerization of Primary Allylic Alcohols under Mild Reaction Conditions. Tetrahedron Lett 2009, 50 (28), 4141-4144. https://doi.org/10.1016/J.TETLET.2009.04.130.
(30) Mantilli, L.; Gérard, D.; Torche, S.; Besnard, C.; Mazet, C. Iridium-Catalyzed Asymmetric Isomerization of Primary Allylic Alcohols. Angewandte Chemie - International Edition 2009, 48 (28), 5143-5147. https://doi.org/10.1002/ANIE.200901863.
(31) Mantilli, L.; Mazet, C. Expanded Scope for the Iridium-Catalyzed Asymmetric Isomerization of Primary Allylic Alcohols Using Readily Accessible Second-Generation Catalysts. Chemical Communications 2010, 46 (3), 445-447. https://doi.org/10.1039/B920342G.
(32) Bricout, H.; Carpentier, J. F.; Mortreux, A. Nickel vs. Palladium Catalysts for Coupling Reactions of Allyl Alcohol with Soft Nucleophiles: Activities and Deactivation Processes. J Mol Catal A Chem 1998, 136 (3), 243-251. https://doi.org/10.1016/S1381-1169(98)00067-3.
(33) Corain, B.; Puosi, G. Isomerization of Olefins and Substituted Olefins Catalyzed by Nickel Complexes. J Catal 1973, 30 (3), 403-408. https://doi.org/10.1016/0021-9517(73)90156-5.
(34) Motherwell, W. B.; Sandham, D. A. Observations on a Nickel-Catalyzed Isomerization of Allylic Lithium Alkoxides. Tetrahedron Lett 1992, 33 (41), 6187-6190. https://doi.org/10.1016/S0040-4039(00)60039-4.
(35) Dedieu, M.; Pascal, Y. L. Transformations d/Alcools et de Glycols α-Ethyleniques Catalysees Par Du Chlorure de Ruthenium. Journal of Molecular Catalysis 1980, 9 (1), 59-70. https://doi.org/10.1016/0304-5102(80)85034-6.
(36) Arai, N.; Sato, K.; Azuma, K.; Ohkuma, T. Enantioselective Isomerization of Primary Allylic Alcohols into Chiral Aldehydes with the Tol-Binap/Dbapen/Ruthenium(II) Catalyst. Angewandte Chemie - International Edition 2013, 52 (29), 7500-7504. https://doi.org/10.1002/ANIE.201303423.
(37) Smadja, W.; Ville, G.; Georgoulis, C. RuCl3-NaoH as a Reagent for Chirality Transfer. Synthesis of Chiral β-Deuteriated Ketones by Asymmetric Induction. Journal of the Chemical Society - Series Chemical Communications 1980, No. 13, 594-595. https://doi.org/10.1039/C39800000594.
(38) Bizet, V.; Pannecoucke, X.; Renaud, J. L.; Cahard, D. Ruthenium-Catalyzed Redox Isomerization of Trifluoromethylated Allylic Alcohols: Mechanistic Evidence for an Enantiospecific Pathway. Angewandte Chemie - International Edition 2012, 51 (26), 6467-6470. https://doi.org/10.1002/ANIE.201200827.
(39) Ito, M.; Kitahara, S.; Ikariya, T. Cp*Ru(PN) Complex-Catalyzed Isomerization of Allylic Alcohols and Its Application to the Asymmetric Synthesis of Muscone. J Am Chem Soc 2005, 127 (17), 6172-6173. https://doi.org/10.1021/JA050770G/SUPPL_FILE/JA050770GSI20050323_125728.PDF.
(40) Doppiu, A.; Salzer, A. A New Roate to Cationic Half-Sandwich Ruthenium(II) Complexes with Chiral Cyclopentadienylphosphane Ligands. Eur J Inorg Chem 2004, No. 11, 2244-2252. https://doi.org/10.1002/EJIC.200300934.
(41) Goetz, R. W.; Orchin, M. The Isomerization of Allyl Alcohols with Cobalt Hydrocarbonyl. J Am Chem Soc 1963, 85 (10), 1549-1550. https://doi.org/10.1021/JA00893A046.
(42) Larionov, E.; Lin, L.; Guénée, L.; Mazet, C. Scope and Mechanism in Palladium-Catalyzed Isomerizations of Highly Substituted Allylic, Homoallylic, and Alkenyl Alcohols. J Am Chem Soc 2014, 136 (48), 16882-16894. https://doi.org/10.1021/JA508736U/SUPPL_FILE/JA508736U_SI_004.CIF.
(43) Isler, O.; Huber, W.; Ronco, A.; Kofler, M. Synthese Des Vitamin A. Helv Chim Acta 1947, 30 (6), 1911-1927. https://doi.org/10.1002/HLCA.19470300666.
(44) Semikolenov, V. A.; Ilyna, I. I.; Maksimovskaya, R. I. Linalool to Geraniol/Nerol Isomerization Catalyzed by (RO)3VO Complexes: Studies of Kinetics and Mechanism. J Mol Catal A Chem 2003, 204-205, 201-210. https://doi.org/10.1016/S1381-1169(03)00299-1.
(45) Belgacem, J.; Kress, J.; Osborn, J. A. Selected NMR Data (CD3CN, Ppm) Are as Follows, La: 6.18 ( CMe2). 3a: 5.66 (t, 1 , 3/Hh = 9.5 Hz, =CH), 4.78 (d, 2, 3yHH = 9.5 Hz, CH2), 1.75 and 1.68 (2 s, 6 H, =CMe2). J. Am. Chem. Soc 1992, 114 (4), 1021.
(46) Belgacem, J.; Kress, J.; Osborn, J. A. Catalytic Oxidation and Ammoxidation of Propylene. Modelling Studies on Well-Defined Molybdenum Complexes. Journal of Molecular Catalysis 1994, 86 (1-3), 267-285. https://doi.org/10.1016/0304-5102(93)E0160-I.
(47) Bellemin-Laponnaz, S.; Gisie, H.; le Ny, J. P.; Osborn, J. A. Mechanistic Insights into the Very Efficient [ReO3OSiR3]-Catalyzed Isomerization of Allyl Alcohols. Angewandte Chemie International Edition in English 1997, 36 (9), 976-978. https://doi.org/10.1002/ANIE. 199709761.
(48) Phane Bellemin-Laponnaz, S. Â.; Jean, ]; le Ny, P.; Dedieu, A. Mechanism of the Allylic Rearrangement of Allyloxo Metal Oxo Complexes: An Ab Initio Theoretical Investigation. https://doi.org/10.1002/(SICI)1521-3765(19990104)5:1.
(49) Volchkov, I.; Lee, D. Recent Developments of Direct Rhenium-Catalyzed [1,3]-Transpositions of Allylic Alcohols and Their Silyl Ethers. Chem Soc Rev 2014, 43 (13), 4381-4394. https://doi.org/10.1039/C4CS00036F.
(50) Belgacem, J.; Kress, J.; Osborn, J. A. Catalytic Oxidation and Ammoxidation of Propylene. Modelling Studies on Well-Defined Molybdenum Complexes. Journal of Molecular Catalysis 1994, 86 (1-3), 267-285. https://doi.org/10.1016/0304-5102(93)E0160-I.
(51) HosogaiTakeo; FujitaYoshiji; NinagawaYoichi; NishidaTakashi. SELECTIVE ALLYLIC REARRANGEMENT WITH TUNGSTEN CATALYST. http://dx.doi.org/10.1246/cl.1982.357 2006, 11 (3), 357-360. https://doi.org/10.1246/CL.1982.357.
(52) Chabardes, P.; Kuntz, E.; Varagnat, J. Use of Oxo-Metallic Derivatives in Isomerisation: Reactions of Unsaturated Alcohols. Tetrahedron 1977, 33 (14), 1775-1783. https://doi.org/10.1016/0040-4020(77)84059-3.
(53) Akai, S.; Tanimoto, K.; Kanao, Y.; Egi, M.; Yamamoto, T.; Kita, Y. A Dynamic Kinetic Resolution of Allyl Alcohols by the Combined Use of Lipases and [VO(OSiPh3)3]. Angewandte Chemie International Edition 2006, 45 (16), 2592-2595. https://doi.org/10.1002/ANIE.200503765.
(54) Cadierno, V.; Crochet, P.; Gimeno, J. Ruthenium-Catalyzed Isomerizations of Allylic and Propargylic Alcohols in Aqueous and Organic Media: Applications in Synthesis. Synlett 2008, No. 8, 1105-1124. https://doi.org/10.1055/S-2008-1072593.
(55) Jacob, J.; Espenson, J. H.; Jensen, J. H.; Gordon, M. S. 1,3-Transposition of Allylic Alcohols Catalyzed by Methyltrioxorhenium. Organometallics 1998, 17 (9), 1835-1840. https://doi.org/10.1021/OM971115N/SUPPL_FILE/OM1835.PDF.
(56) Wang, G.; Jimtaisong, A.; Luck, R. L. Allylic Alcohol Isomerization and Mechanistic Considerations with CH 3ReO 3. Organometallics 2004, 23 (19), 4522-4525. https://doi.org/10.1021/OM049669V/SUPPL_FILE/OM049669VSI20040702_040820.PDF.
(57) Herrmann, A. T.; Saito, T.; Stivala, C. E.; Tom, J.; Zakarian, A. Regio- and Stereocontrol in Rhenium-Catalyzed Transposition of Allylic Alcohols. J Am Chem Soc 2010, 132 (17), 5962-5963. https://doi.org/10.1021/JA101673V/SUPPL_FILE/JA101673V_SI_001.PDF.
(58) Smith, M. B.; March, J. Oxidations and Reductions. March's Advanced Organic Chemistry 2007, 1703-1869. https://doi.org/10.1002/9780470084960.CH19.
(59) The Chemistry of Fragrances. The Chemistry of Fragrances 2006. https://doi.org/10.1039/9781847555342.
(60) Johnson, T. C.; Morris, D. J.; Wills, M. Hydrogen Generation from Formic Acid and Alcohols Using Homogeneous Catalysts. Chem Soc Rev 2009, 39 (1), 81-88. https://doi.org/10.1039/B904495G.
(61) Dobereiner, G. E.; Crabtree, R. H. Dehydrogenation as a Substrate-Activating Strategy in Homogeneous Transition-Metal Catalysis. Chem Rev 2010, 110 (2), 681-703. https://doi.org/10.1021/CR900202J/ASSET/CR900202J.FP.PNG_V03.
(62) Gunanathan, C.; Milstein, D. Applications of Acceptorless Dehydrogenation and Related Transformations in Chemical Synthesis. Science (1979) 2013, 341 (6143). https://doi.org/10.1126/SCIENCE.1229712.
(63) Bilis, G.; Louloudi, M. The Catalytic Function of Nonheme Iron (III) Complex for Hydrocarbon Oxidation. Bioinorg Chem Appl 2010, 2010. https://doi.org/10.1155/2010/861892.
(64) Baratta, W.; Bossi, G.; Putignano, E.; Rigo, P. Pincer and Diamine Ru and Os Diphosphane Complexes as Efficient Catalysts for the Dehydrogenation of Alcohols to Ketones. Chemistry - A European Journal 2011, 17 (12), 3474-3481. https://doi.org/10.1002/CHEM.201003022.
(65) Ngo, A. H.; Adams, M. J.; Do, L. H. Selective Acceptorless Dehydrogenation and Hydrogenation by Iridium Catalysts Enabling Facile Interconversion of Glucocorticoids. Organometallics 2014, 33 (23), 6742-6745. https://doi.org/10.1021/OM5010258/SUPPL_FILE/OM5010258_SI_001.PDF.
(66) Liu, Y.; Xie, A.; Li, J.; Xu, X.; Dong, W.; Wang, B. Heterocycle-Substituted Tetrazole Ligands for Copper-Catalysed Aerobic Oxidation of Alcohols. Tetrahedron 2014, 70 (52), 9791-9796. https://doi.org/10.1016/J.TET.2014.11.015.
(67) Moody, C. J.; Palmer, F. N. Dirhodium(II) Carboxylate-Catalysed Oxidation of Allylic and Benzylic Alcohols. Tetrahedron Lett 2002, 43 (1), 139-141. https://doi.org/10.1016/S0040-4039(01)02048-2.
(68) Hanson, S. K.; Wu, R.; Silks, L. A. P. Mild and Selective Vanadium-Catalyzed Oxidation of Benzylic, Allylic, and Propargylic Alcohols Using Air. Org Lett 2011, 13 (8), 1908-1911. https://doi.org/10.1021/OL103107V/SUPPL_FILE/OL103107V_SI_001.PDF.
(69) Schultz, M. J.; Hamilton, S. S.; Jensen, D. R.; Sigman, M. S. Development and Comparison of the Substrate Scope of Pd-Catalysts for the Aerobic Oxidation of Alcohols. Journal of Organic Chemistry 2005, 70 (9), 3343-3352. https://doi.org/10.1021/JO0482211/SUPPL_FILE/JO0482211SI20041110_020546.PDF.
(70) Ménage, S.; Galey, J. B.; Dumats, J.; Hussler, G.; Seité, M.; Luneau, I. G.; Chottard, G.; Fontecave, M. O2 Activation and Aromatic Hydroxylation Performed by Diiron Complexes. J Am Chem Soc 1998, 120 (51), 13370-13382. https://doi.org/10.1021/JA981123A/SUPPL_FILE/JA981123A_S.PDF.
(71) Tshuva, E. Y.; Lippard, S. J. Synthetic Models for Non-Heme Carboxylate-Bridged Diiron Metalloproteins: Strategies and Tactics. Chem Rev 2004, 104 (2), 987-1012. https://doi.org/10.1021/CR020622Y/ASSET/CR020622Y.FP.PNG_V03.
(72) Akbayeva, D. N.; Gonsalvi, L.; Oberhauser, W.; Peruzzini, M.; Vizza, F.; Brüggeller, P.; Romerosa, A.; Sava, G.; Bergamo, A. Synthesis, Catalytic Properties and Biological Activity of New Water Soluble Ruthenium Cyclopentadienyl PTA Complexes [(C5R5)RuCl(PTA)2] (R = H, Me; PTA = 1,3,5-Triaza-7-Phosphaadamantane). Chemical Communications 2003, 2 (2), 264-265. https://doi.org/10.1039/B210102E.
(73) Scalambra, F.; Serrano-Ruiz, M.; Romerosa, A. Water and Catalytic Isomerization of Linear Allylic Alcohols by [RuCp(H 2 O-KO)(PTA) 2 ] + (PTA = 1,3,5-Triaza-7-Phosphaadamantane). Dalton Transactions 2017, 46 (18), 5864-5871. https://doi.org/10.1039/C6DT04262G.
(74) Serrano-Ruiz, M.; Lorenzo-Luis, P.; Romerosa, A.; Mena-Cruz, A. Catalytic Isomerization of Allylic Alcohols in Water by [RuClCp(PTA)2], [RuClCp(HPTA)2]Cl2·2H2O, [RuCp(DMSO-KS)(PTA)2]Cl, [RuCp(DMSO-KS)(PTA)2](OSO2CF3) and [RuCp(DMSO-KS)(HPTA)2]Cl3·2H2O. Dalton Transactions 2013, 42 (21), 7622. https://doi.org/10.1039/c3dt32998d.
(75) González, B.; Lorenzo-Luis, P.; Serrano-Ruiz, M.; Papp, É.; Fekete, M.; Csépke, K.; Ősz, K.; Kath6, Á.; Joó, F.; Romerosa, A. Catalysis of Redox Isomerization of Allylic Alcohols by [RuClCp(MPTA)2](OSO2CF3)2 and [RuCp(MPTA)2(OH2-KO)](OSO2CF3)3·(H2O)(C4H10O)0.5. Unusual Influence of the PH and Interaction of Phosphate with Catalyst on the Reaction Rate. *J Mol Catal A Chem* **2010,** *326* (1-2), 15-20. https://doi.org/10.1016/j.molcata.2010.05.003.
(76) Scalambra, F.; López-Sanchez, B.; Romerosa, A. Good Isomerization of 2-Cyclohexenol by Two Ru( <scp>ii</Scp> ) Complexes, Synthesis and Characterization of a Reaction Intermediate. Dalton Transactions 2018, 47 (46), 16398-16402. https://doi.org/10.1039/C8DT02560F.
(77) Scalambra, F.; Lopez-Sanchez, B.; Holzmann, N.; Bernasconi, L.; Romerosa, A. Steps Ahead in Understanding the Catalytic Isomerization Mechanism of Linear Allylic Alcohols in Water: Dynamics, Bonding Analysis, and Crystal Structure of an η 2 -Allyl-Intermediate. Organometallics 2020, 39 (24), 4491-4499. https://doi.org/10.1021/acs.organomet.0c00585.
(78) Bakthavatsalam, N. Semiochemicals. Ecofriendly Pest Management for Food Security 2016, 563-611. https://doi.org/10.1016/B978-0-12-803265-7.00019-1.
(79) Six, D. L.; Bracewell, R. Dendroctonus. Bark Beetles: Biology and Ecology of Native and Invasive Species 2015, 305-350. https://doi.org/10.1016/B978-0-12-417156-5.00008-3.
(80) Chen, G.; Song, Y.; Wang, P.; Chen, J.; Zhang, Z.; Wang, S.; Huang, X.; Zhang, Q. H. Semiochemistry of Dendroctonus Armandi Tsai and Li (Coleoptera: Curculionidae: Scolytinae): Both Female-Produced Aggregation Pheromone and Host Tree Kairomone Are Critically Important. Chemoecology 2015, 25 (3), 135-145. https://doi.org/10.1007/S00049-014-0182-1/FIGURES/4.
(81) Isitt, R. L.; Bleiker, K. P.; Pureswaran, D. S.; Hillier, N. K.; Huber, D. P. W. Local, Geographical, and Contextual Variation in the Aggregation Pheromone Blend of the Spruce Beetle, Dendroctonus Rufipennis (Coleoptera: Curculionidae). J Chem Ecol 2020, 46 (5-6), 497-507. https://doi.org/10.1007/S10886-020-01185-3/FIGURES/5.
(82) Isitt, R. L.; Bleiker, K. P.; Pureswaran, D. S.; Hillier, N. K.; Huber, D. P. W. The Effect of Feeding and Mate Presence on the Pheromone Production of the Spruce Beetle (Coleoptera: Curculionidae). Environ Entomol 2018, 47 (5), 1293-1299. https://doi.org/10.1093/EE/NVY092.
(83) Johnston, B. D.; Morgan, B.; Oehlschlager, A. C.; Ramaswamy, S. A Convenient Synthesis of Both Enantiomers of Seudenol and Their Conversion to 1-Methyl-2-Cyclohexen-1-Ol (Mcol). Tetrahedron Asymmetry 1991, 2 (5), 377-380. https://doi.org/10.1016/S0957-4166(00)82123-0.
(84) Ross, D. W.; Datermanl, G. E. Response of Dendroctonus Pseudotsugae (Coleoptera: Scolytidae) and Thanasimus Undatulus (Coleoptera: Cleridae) to Traps with DUJerentSenriochenricms. J. Econ.Entomol 1995, 88, 106-111.
(85) Huber, D. P. W.; Borden, J. H. Angiosperm Bark Volatiles Disrupt Response of Douglas-Fir Beetle, Dendroctonus Pseudotsugae, to Attractant-Baited Traps. J Chem Ecol 2001, 27 (2), 217-233. https://doi.org/10.1023/A:1005668019434.
(86) Mori, K.; Hazra, B. G.; Pfeiffer, R. J.; Gupta, A. K.; Lindgren, B. S. Synthesis and Bioactivity of Optically Active Forms of 1-Methyl-2-Cyclohexen-1-Ol. An Aggregation Pheromone of Dendroctonus Pseudotsugae. Tetrahedron 1987, 43 (10), 2249-2254. https://doi.org/10.1016/S0040-4020(01)86808-3.
(87) Ryker, L. C.; Libbey, L. M.; Rudinsky, J. A. Comparison of Volatile Compounds and Stridulation Emitted by the Douglas-Fir Beetle from Idaho and Western Oregon Populations. Environ Entomol 1979, 8 (5), 789-798. https://doi.org/10.1093/EE/8.5.789.
(88) Pureswaran, D. S.; Borden, J. H. New Repellent Semiochemicals for Three Species of Dendroctonus (Coleoptera: Scolytidae). CHEMOECOLOGY 2004 14:2 2004, 14 (2), 67-75. https://doi.org/10.1007/S00049-003-0260-2.
(89) Rudinskv, J. A.; Kline, " L N; Diekman, J. D. Response-Inhibition by Four Analogues of MCH, an Antiaggregative Pheromone of the Douglas-Fir Beetle 1.
(90) Tsuboi, T.; Takaguchi, Y.; Tsuboi, S. Photoinduced Isomerization of Allyl Alcohols to Carbonyl Compounds Using Dendrimer Disulfide as Catalyst. Heteroatom Chemistry 2009, 20 (1), 1-11. https://doi.org/10.1002/HC.20504.
(91) Matsubara, S.; Okazoe, T.; Oshima, K.; Takai, K.; Nozaki, H. Isomerization of Allylic Alcohols Catalyzed by Vanadium or Molybdenum Complexes. http://dx.doi.org/10.1246/bcsj.58.844 2006, 58 (3), 844-849. https://doi.org/10.1246/BCSJ.58.844.
(92) Li, P. F.; Wang, H. L.; Qu, J. 1,n-Rearrangement of Allylic Alcohols Promoted by Hot Water: Application to the Synthesis of Navenone B, a Polyene Natural Product. Journal of Organic Chemistry 2014, 79 (9), 3955-3962. https://doi.org/10.1021/JO5004086/SUPPL_FILE/JO5004086_SI_001.PDF.
(93) Frost, B. J.; Mebi, C. A. Aqueous Organometallic Chemistry: Synthesis, Structure, and Reactivity of the Water-Soluble Metal Hydride CpRu(PTA) 2H. Organometallics 2004, 23 (22), 5317-5323. https://doi.org/10.1021/OM049501.
(94) Mebi, C. A.; Nair, R. P.; Frost, B. J. PH-Dependent Selective Transfer Hydrogenation of α,β-Unsaturated Carbonyls in Aqueous Media Utilizing Half-Sandwich Ruthenium(II) Complexes. Organometallics 2007, 26 (2), 429-438. https://doi.org/10.1021/OM060892X/SUPPL_FILE/OM060892XSI20060927_124128.CIF.
(95) López-Sánchez, B.; Bohome-Espinosa, A. B.; Scalambra, F.; Romerosa, A. Ru Complexes Containing N-Methyl-1,3,5-Triaza-7-Phosphaadamantane (MPTA) as Catalysts for the Isomerization of 2-Cyclohexen-1-Ol. Appl Organomet Chem 2022, e6971. https://doi.org/10.1002/AOC.6971.
(96) Mebi, C. A.; Frost, B. J. Effect of PH on the Biphasic Catalytic Hydrogenation of Benzylidene Acetone Using CpRu(PTA) 2H. Organometallics 2005, 24 (10), 2339-2346. https://doi.org/10.1021/OM048987R/SUPPL_FILE/OM048987RSI20050310_030915.PDF.
(97) Â Marko Â, I. E.; Gautier, A.; Tsukazaki, M.; Llobet, A.; Plantalech-Mir, E.; Urch, C. J.; Brown, S. M. Novel and Efficient Isomerization of Allylic Alcohols Promoted by a Tetrapropylammonium Perruthenate Catalyst∗∗. https://doi.org/10.1002/(SICI)1521-3773(19990712)38:13/14.

## Claims

1. A process for the catalytic isomerization, oxidation, 1,3-transposition and/or etherification of 3-methyl-2-cyclohexen-1-ol to 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or any mixture thereof, comprising:
a. providing a starting material or composition comprising 3-methyl-2-cyclohexen-1-ol;
b. contacting the starting material or composition with an aqueous and/or organic media; and
c. carrying out a reaction to produce 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or any mixture thereof; and
d. optionally recovering the 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or any mixture thereof;
wherein the reaction of step c) is performed in the aqueous or organic media in contact with the starting material or composition of step b), during a time interval of between 1 minute and 7 days, preferably between 1 hour and 3 days, and wherein the reaction takes place at a pressure within the range of 10⁻² and 10 atmospheres (atm), preferably 1 atm, and at a temperature between 0.1°C and 150°C.

2. The process of claim 1, wherein step b) is carried out by contacting the starting material or composition with the aqueous and/or organic media at a molar ratio of the aqueous and/or organic media to 3-methyl-2-cyclohexen-1-ol between 1:0.01 and 1:10000 v/v.

3. The process of any one of claims 1 or 2, wherein the aqueous and/or organic media is an aqueous media, preferably water, and the reaction of step c) takes place under aerobic conditions or under an inert atmosphere such as a nitrogen atmosphere, at a temperature between 30°C and 100°C, and the concentration of 3-methyl-2-cyclohexen-1-ol used in contacting step b) is greater than 0.05 mM.

4. The process according to claim 3, wherein the concentration of 3-methyl-2-cyclohexen-1-ol used in contacting step b) is between 0.1 mM and 10 mM, preferably 0.9 mM.

5. The process according to claim 3, wherein the concentration of 3-methyl-2-cyclohexen-1-ol used in contacting step b) is between 0.05 mM and 0.5 mM, preferably 0.2 mM.

6. The process according to any one of claims 1 or 2, where the process comprises:
a. providing a starting material or composition comprising 3-methyl-2-cyclohexen-1-ol;
b. contacting the starting material or composition with a catalyst in an aqueous or organic media;
c. carrying out a reaction to produce 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or any mixture thereof; and
d. optionally recovering the 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or any mixture thereof;
wherein the reaction of step c) is performed in the aqueous or organic media in contact with the starting material or composition of step b), during a time interval of between 1 minute and 7 days, preferably between 1 hour and 3 days, and wherein the reaction takes place at a pressure within the range of 10⁻² and 10 atmospheres (atm), preferably 1 atm, and at a temperature between 0.1°C and 150°C.

7. The process according to claim 6, wherein the catalyst has a chemical structure of formula I: 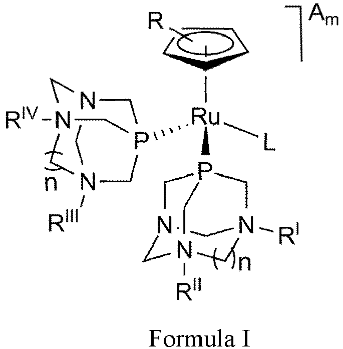 wherein:
- L = fluoride, chloride, bromide, iodide, carbonyl, cyanide, hydride; azide, pyridine, pyrazine, pyrimidine, bipyridine, N-heterocyclic carbenes; alcohols such as methanol, isopropanol, ethanol, tert-butanol, n-butanol, and their alkoxides; oxobases such as phosphate, carbonate, sulfate, formate, and their acid derivatives; allylic alcohol η²-complexes;
- R is H or a methyl group (CH₃);
- RI, RII, RIII, RIV are independently selected from an oxygen atom, H, a methyl group (CH₃), a (CH₂)ₙ-CH₃ (wherein n is 1 - 20), an acetyl, allyl, butyl, phenyl, benzyl, nitrobenzyl, or dichloroallyl group;
- n is 0-1;
- wherein A is preferably selected from chloride, iodide, phosphate, triflate, sulfate, bromide, tetrafluoroborate, hexafluoroborate, tetrafluoromethane, tetrafluoroammonium, BArF; and
- m is 0 - 3.

8. The process according to any one of claims 6 to 7, wherein the catalyst is selected from the list consisting of the metal complexes [RuClCp(PTA)₂] (1) or [RuCp(OH₂)(PTA)₂]CF₃SO₃ (2) (PTA = 1,3,5-triaza-7-phosphaadamantane).

9. The process according to claim 8, wherein the catalyst is [RuCp(OH₂)(PTA)₂]CF₃SO₃ (2) and the reaction takes place under an inert atmosphere such as a nitrogen atmosphere, at a temperature between 30°C and 100°C, and the concentration of the catalyst used during step b) is equal or greater than 0.6 mol%, preferably equal or greater than 0.7 mol%, preferably equal or greater than 0.8 mol%, preferably equal or greater than 0.9 mol%, preferably equal or greater than 1 mol%, preferably equal or greater than 5 mol%, preferably equal or greater than 10 mol%.

10. The process according to claim 8, wherein the catalyst is selected from the list consisting of the metal complexes [RuClCp(PTA)₂] (1) or [RuCp(OH₂)(PTA)₂]CF₃SO₃ (2) (PTA = 1,3,5-triaza-7-phosphaadamantane), and the reaction takes place under aerobic conditions and the concentration of the catalyst used during step b) is between 0.05 and 0.5 mol%, preferably about 0.1 mol%, and the aqueous media is water.

11. The process according to claim 8, wherein the catalyst is [RuClCp(PTA)₂] (1), and the reaction takes place under an inert atmosphere such as a nitrogen atmosphere, at a temperature between 30°C and 100°C, preferably about 70°C, and the concentration of the catalyst used during step b) is equal or greater than 0.6 mol%, preferably equal or greater than 0.7 mol%, preferably equal or greater than 0.8 mol%, preferably equal or greater than 0.9 mol%, preferably equal or greater than 1 mol%, preferably equal or greater than 5 mol%, preferably equal or greater than 10 mol%.

12. The process according to claim 11, wherein the aqueous or organic media used during step b) is an aqueous media such as water or an organic solvent or a mixture of water and an organic solvent such as cyclohexane, toluene, octane, heptane, hexane, pentane, benzene, xylene, or fluorated solvents, wherein preferably the mixture of water to organic solvent is present at a ratio of from 0.01:1 to 100:1 v/v.

13. The process of any one of claims 1 or 12, wherein the reaction process takes place at a pH between 3 and 9, preferably at about 8 or at about 4.5.

14. A composition comprising 1-methyl-2-cyclohexen-1-ol, 3-methyl-2-cyclohexenone, 3-methylcyclohexanone, 1-methyl-3-(3-methyl-2-cyclohexen-1-yl)oxycyclohexene or a mixture thereof obtained by the process of any of claims 1 to 13.

15. Use of the composition of claim 14 for the control of Douglas-fir beetle.
